(19) Europäisches Patentamt
European Patent Office
Office européen des brevets

(11) Publication number: **0 216 973 B1**

(12) **EUROPEAN PATENT SPECIFICATION**

(45) Date of publication of the patent specification: **23.11.89**

(51) Int. Cl.⁴: **C07D 327/00**, G03C 1/04

(21) Application number: **85401850.4**

(22) Date of filing: **24.09.85**

(54) **Photographic elements and compositions containing cyclic thioethers.**

(43) Date of publication of application: **08.04.87 Bulletin 87/15**

(45) Publication of the grant of the patent: **23.11.89 Bulletin 89/47**

(84) Designated Contracting States: **BE CH DE FR GB LI NL**

(56) References cited:
**GB-A- 942 865**

**J.S. BRADSHAW et al. J. Heterocyclic Chem. 11 (1) 1974 p. 45 ff.**
**J.S. BRADSHAW et al. J. Heterocyclic Chem. 10 (1) 1973 p.1 ff**
**L. MORTILLARD et al. J. Chem. Soc. C 4 1966 p. 428 ff**
**C.J. PEDERSEN J. Org. Chem. 36 (2) 1971 p. 254 ff.**

**The file contains technical information submitted after the application was filed and not included in this specification**

(73) Proprietor: **EASTMAN KODAK COMPANY (a New Jersey corporation), 343 State Street, Rochester New York 14650(US)**

(72) Inventor: **Benard, Réjane, KODAK-PATHE 30 rue des Vignerons BP 60, F-94302 Vincennes Cedex(FR)**
Inventor: **Friour, Gérard, KODAK-PATHE 30 rue des Vignerons BP 60, F-94302 Vincennes Cedex(FR)**
Inventor: **Riveccie, Marcel, KODAK-PATHE 30 rue des Vignerons BP 60, F-94302 Vincennes Cedex(FR)**

(74) Representative: **Parent, Yves et al, Kodak-Pathé Département Brevets et Licences Centre de Recherches et de Technologie Zone Industrielle, F-71102 Chalon-sur-Saône Cédex(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The present invention relates to photographic elements and compositions containing cyclic thioethers.

The published prior art shows that the addition of various thioethers has been recommended in various steps of the preparation of silver halide emulsions, as well as in photographic processing solutions.

It is known in particular that many thioethers are useful to improve the sensitivity of the emulsions sensitized with sulfur compounds and gold salts.

Such a use of thioethers is described for example in French patent 1,004,983 and in U.S. patents 3,021,215 ; 3,046,129 ; 3,046,132 ; 3,046,133 and 3,046,134. In French patent 1,004,983 it is proposed to increase the speed of an emulsion by adding β-aminoethyl sulfide during chemical ripening. According to U.S. patent 3,021,215, the chemical sensitization of an emulsion can be improved by the addition of polythiaalkyleneglycols ; for the same reason, U.S. patent 3,046,129 recommends the use of polythioethers having the formula $-(R-S)_x$, wherein x is greater than 3. Other compounds comprising thioether groups, added at the time of chemical ripening, or before emulsion coating, are described in French patents 1,271,381 ; 1,280,802 and 1,541,980. French patent 1,377,252 describes cyclic thioethers useful as sensitizers.

However, while these thioethers have a certain effectiveness for increasing the sensitivity of the emulsions, they may correlatively produce an increase of the fog. This risk is inherent in most sensitization techniques and it is therefore interesting in each specific case to look for means that permit to minimize it.

U.S. patent 3,625,697 describes compounds having the formula:

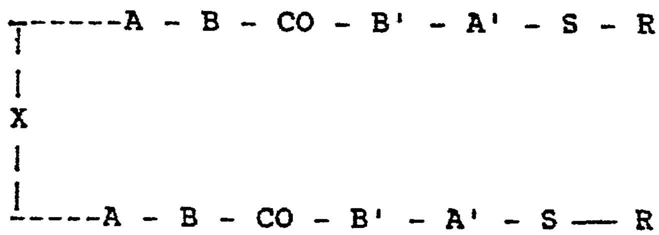

wherein A and A' are alkylene or polyalkyleneether groups, provided A and A' are not at the same time polyalkyleneether.

B and B' are NH- or 0-, provided they are not -0- at the same time.

R is a lower alkyl group, a phenyl, aralkyl or $-CH_2-COOR'$ and X is a divalent group having the formula -S-, -O-, alkyl, aralkyl or amine.

These compounds added to emulsions increase the sensitivity without increasing the fog.

More recently, U.S. patent 4,276,374 describes the use of polyethers having the formula:

$$R^1-(X-R^3)_m-X-R^2$$

wherein X is S or O, $R^1$ and $R^2$ are alkyl groups bearing various substituents such as OH, COOH, amino, etc., $R^3$ is an alkylene group, m represents 0 or an integer of 1 to 4, during precipitation or as addenda either of finishing or of melting.

From the results shown, these thioethers lead to a substantial sensitivity gain without any fog growth.

French patent 2,215,642 describes cyclic compounds containing at least 4 atoms of P, of and N or of S as sensitizers. A compound containing 4 S is cited but the possible use thereof is not illustrated by any example; besides, this compound does not contain any 0.

USSR patent 1,038,919 describes sensitizers which are cyclic thioethers comprising two sulfur atoms and two or four oxygen atoms. Some are precomplexed with $Ag^{++}$ or $Cu^{++}$ ions. None contains three sulfur atoms.

Although in most of these patents, in the general formula of the compound there is provision for the possible presence of 3 and more atoms of S, none discloses nor suggests the increased activity of the compounds comprising at least 3 S in comparison to those that contain 1 or 2 only.

In Journal of Organic Chemistry, Vol 36, No 2, pp 254–257, 1971, are disclosed the complexing properties of a certain number of crown-ethers containing from two to four sulfur atoms. No disclosure is given concerning the photographic properties of such crown-ethers.

The synthesis of crown-ethers containing from one to four sulfur atoms are described in J.S. Bradshaw et al., J. Heterocyclic Chem. 11 (1) 1974, p 45, J.S. Bradshaw et al. J. Heterocyclic Chem. 10 (1) 1973, p 1, L. Mortillard et al. J. Chem. Soc. C.4. (1966) p 428.

There is a continuous concern to seek compounds improving the sensitivity of silver halide emulsions without increasing the fog in an objectionable manner.

The present invention has for its object a photographic element comprising cyclic thioethers, non polymers, comprising at least 3 atoms of sulfur and at least 1 atom of oxygen, each atom of sulfur or oxygen

being separated from each other by a polymethylene group. In a preferred embodiment, sulfur and/or oxygen atoms are separated by bridges $-(CH_2)_n-$, wherein n is an integer equal to 2 or 3.

These compounds are particularly effective as co-sensitizers or development accelerators.

The co-sensitization effect is described in French Patent 1,377,252 cited above. The co-sensitizers have an effect which is added to those which result from the conventional sensitization of photographic emulsions made at an optimum value or near the latter by means of usual chemical sensitizers such as sulfur, selenium or gold compounds.

Various kinds of compounds, incorporated either in the photographic element or in the developer can produce an accelerated development. These compounds are described in C.E. Mees, "The Theory of the Photographic Process" 4th edition, pages 422-426. Thioethers have been described as development accelerators, for example in U.S. Patent 3,201,242 and in Research Disclosure, item 23005, June 1983, as stated above.

It is believed that the effectiveness of thioethers is due to an increased solvation action on silver halides, because they are capable of forming polynuclear complexes with Ag+ ions. These complexes have the characteristics of being thermodynamically stable and kinetically labile, i.e. they are dissociated and recomplexed rapidly, performing the function of Ag+ ion pumps.

This property authorizes a better regulation of sensitization and of chemical ripening.

The compounds for use in photographic elements or compositions according to the invention include in particular the following compounds:

1,4-dioxa-7,10,13-trithiacyclopentadecane

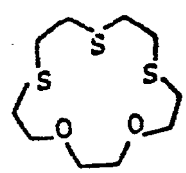

1-oxa-6,9,12-trithiacyclohexadecane

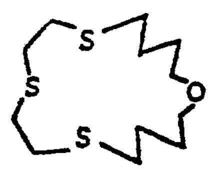

1,7,13-trioxa-4,10,16-trithiacyclooctadecane

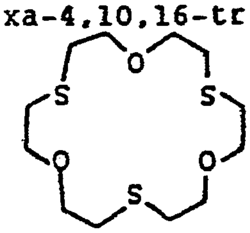

1,4,7-trioxa-10,13,16-trithiacyclooctadecane

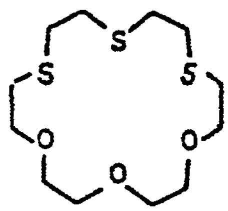

1,4,7,10-tetraoxa-13,16,19-trithia-cycloheneicosane

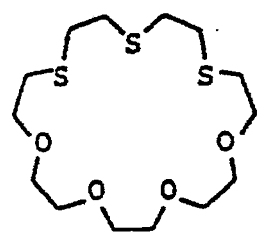

1-10-dioxa-4,7,13,16-tetrathiacyclo-octadecane

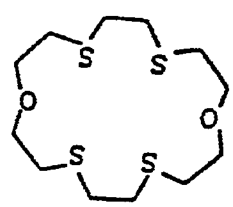

Cyclic thioethers can be prepared according to the invention by synthetic processes described in the literature.

In a general manner these compounds are obtained by reacting an ω-dihalogenated compound with a dithiol.

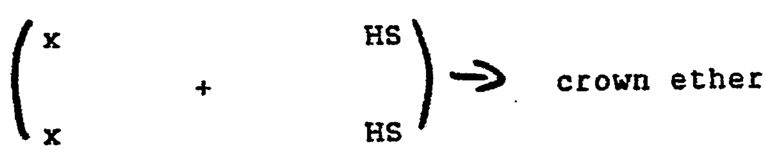


with x = halogen.

The first procedure used, which is described in French Patent 1,377,252, is the following:

3 liters of 50% ethanol are placed in a 4 liter three-necked flask. 10,6 g (0.1 mole) of sodium carbonate $Na_2CO_3$ are added and the mixture is refluxed.

To the mixture is then added the dihalogenated derivative (0.1 mole) and dithiol (0.1 mole) in 2 h.

After refluxing for 48 h, the solvents are evaporated in vacuo and the residue is extracted with chloroform.

The final product is isolated by chromatography on silica gel Merck 60H.

The second procedure given hereafter is described by J. Botter, R.M. Kellog, in J.Org. Chem. 1981, page 4481. After flushing with nitrogen, 1250 ml of dimethylformamide (DMF) are placed in a 2 liter three-necked flask. 35.85 g (0.11 mole) of cesium carbonate $Cs_2CO_3$ are then added.

The temperature of the reaction medium is raised to 80°C and the mixture of dihalogenated derivative (0.1 mole) and dithiol (0.1 mole) in solution in 500 ml of DMF is added in 8 h.

At the end of the addition, the reaction medium is cooled to room temperature, then filtered. The solvents are evaporated in vacuo and the product is then isolated by chromatography on silica gel Merck 60H.

<u>COMPOUND 1:</u>

<u>1,4-dioxa-7,10,13-trithiacyclopentadecane</u>

This compound is obtained by reacting 1,5-dimercapto-3-thiapentane with 1,8-dichloro-3,6-dioxa-octabe in the presence of cesium carbonate in dimethylformamide (DMF).

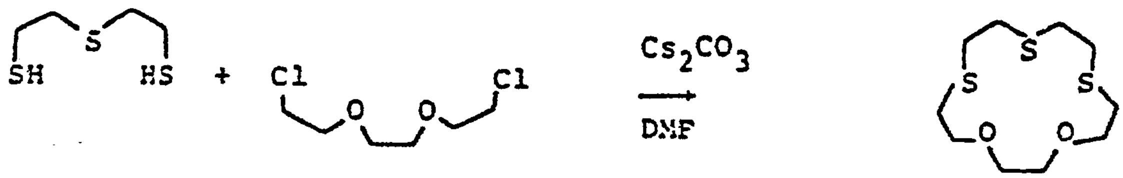


<u>COMPOUND 2:</u>

<u>1-oxa-6,9,12-trithiacyclohexadecane</u>

This compound is prepared by reacting 1,5-dimercapto-3-thiapentane with 1,9-dichloro 5-oxanonane in dimethylformamide in the presence of cesium carbonate.

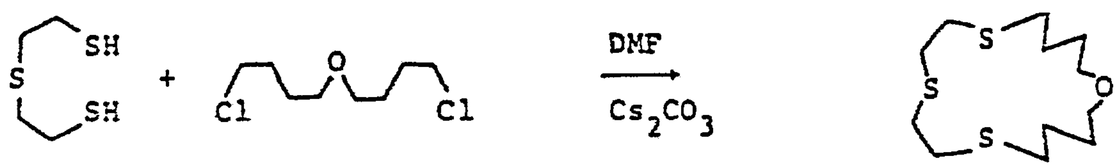


The dihalogenated compound is prepared by reacting $POCl_3$ with THF.

<u>COMPOUND 3:</u>

<u>1,7,13-trioxa 4,10,16-trithiacyclooctadecane</u>

The reaction between sodium sulfide and 2-(2'-chloro-ethoxy)ethanol leads to the formation of 1,11-dihydroxy-3,9-dioxa -6-thiaundecane which is dihalogenated to provide compound A.

The condensation of A with 1,5-dimercapto-3-oxapentane yields compound 3.

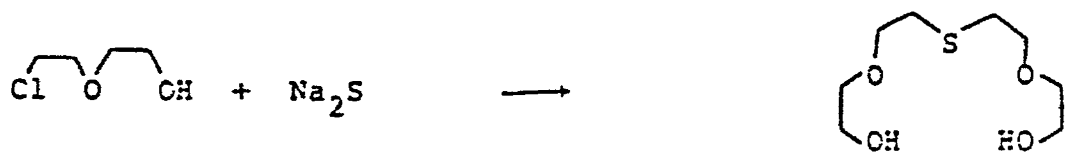


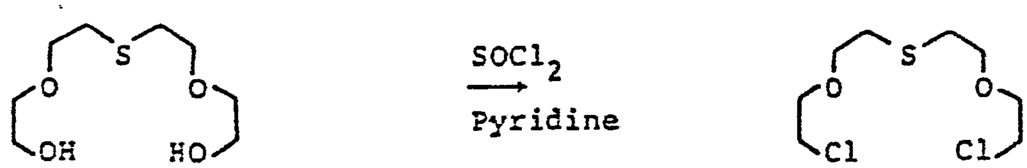


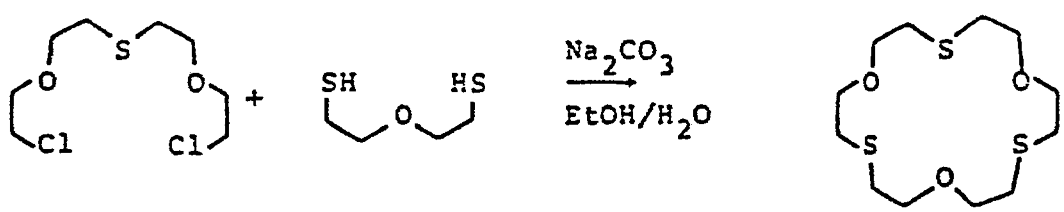


COMPOUND 4:

1,4,7-trioxa-10,13,16-trithiacyclo- octedecane

This compound is obtained by reacting 1,11-dichloro-3,6,9-trioxaundecane with 1,5-dimercapto-3-thiapentane.

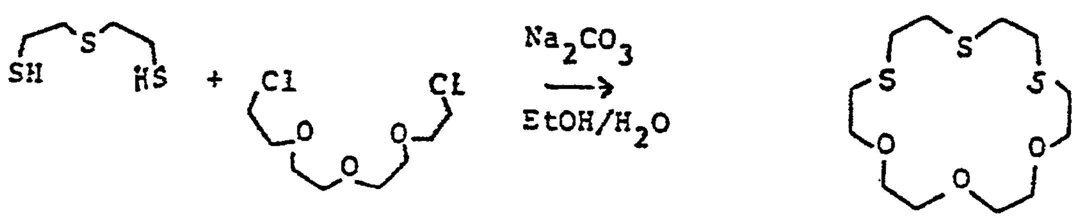


COMPOUND 5:

1,4,7,10-tetraoxa 13,16,19-trithiacycloheneicosane

This compound is obtained by reacting 1,5-dimercapto-3-thiapentane with 1,14-dichloro-3,6,9,12-tetraoxatetradecane

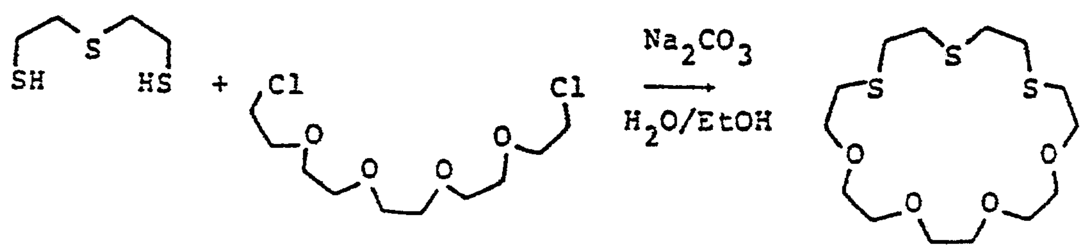

COMPOUND 6:

1,10-dioxa-4,7,13,16-tetrathiacyclooctadecane

The reaction between the disodium salt of ethane dithiol and 2-(2'-chloro-ethoxy)ethanol supplies 1,14-dihydroxy-3,12-dioxa 6,9-dithiatetradecane which is dihalogenated to supply B.

The condensation of B with ethane dithiol yields compound 6.

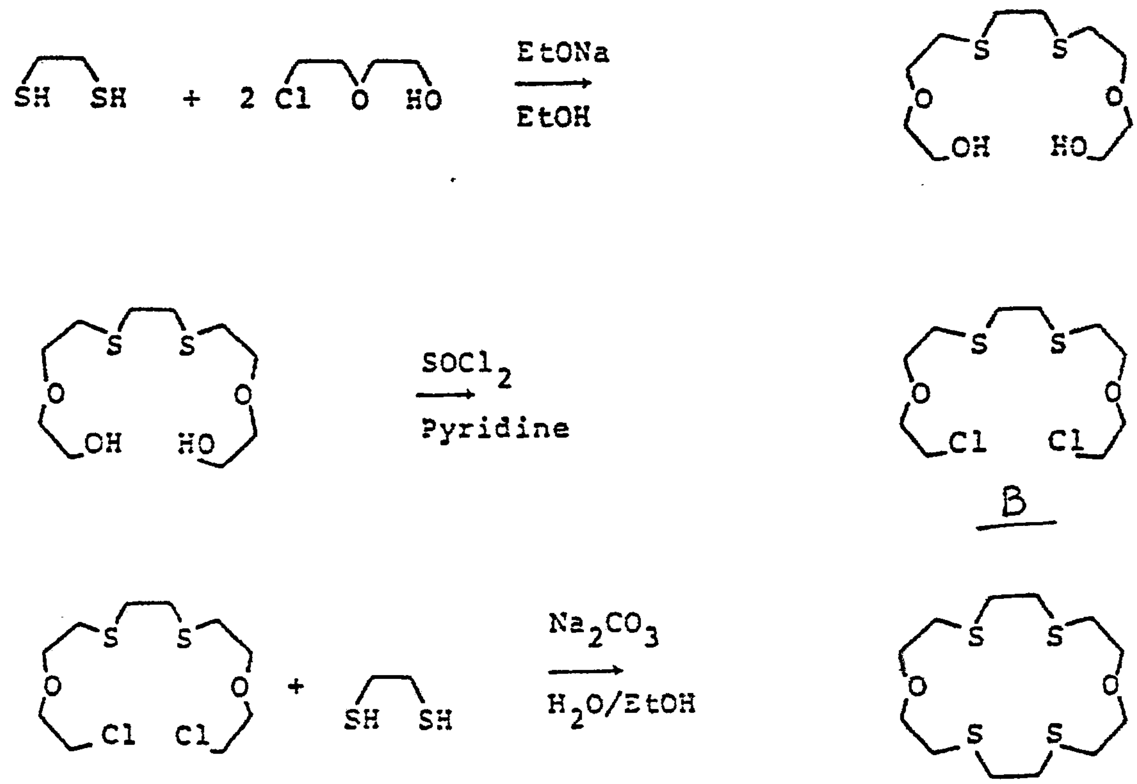


The photographic element according to the invention contains a support and at least one silver halide emulsion layer.

The cyclic thioether as described can be present in a silver halide emulsion layer or in an intermediate layer. It is preferably incorporated in an emulsion layer.

When it is present in an emulsion layer, it can be incorporated at various steps of the preparation of the emulsion.

The preparation of silver halide emulsions is well known and is described, for example, in Research Disclosure, December 1978, item 17643, paragraphs I and II. The emulsions can be chemically sensitized as described in paragraph III of the same publication.

The thioether as described can be incorporated in the chemical sensitization step of the preparation of silver halide emulsions, simultaneously with other chemical sensitizers. Amounts of thioether comprised in a wide range can be used in accordance with the type of emulsion, the thioether selected or the specific effects that are sought but, generally, these amounts are from $5.10^{-5}$ to $1.10^{-3}$ mole/silver mole and preferably from $1.10^{-4}$ to $5.10^{-4}$ mole/silver mole. Preferably, the chemical sensitizers are sulfur and/or selenium compounds and gold compounds. In this case, the cyclic thioether acts as a cosensitizer. In comparison to the thioethers used as cosensitizers according to the prior art, the cyclic thioethers of the present invention give a better sensitivity to the emulsion without any undesirable increase of fog, as shown by the examples below.

According to another embodiment, the thioether compound as described is incorporated during the melting step of the silver halide emulsion. In this case, the amounts are preferably from $1.10^{-4}$ to $5.10^{-4}$ mole/silver mole.

According to another embodiment, the compound is incorporated in an intermediate layer of the photographic element, at a coverage ratio of from $1.10^{-7}$ to $1.10^{-6}$ mg/dm$^2$.

In the last two embodiments, it is assumed that the thioether acts rather as a development accelerator. An increase of the sensitivity is also noted here from the sensitometric results obtained with exposed and processed photographic elements containing a thioether of the prior art.

The silver halide grains can be of any halide composition (e.g., silver bromide, silver bromoiodide, silver chloride, or silver chlorobromoiodide), any size (e.g., coarse or fine), and regular or irregular shape (e.g., spherical, regular cubic, regular octahedral, cubo-octahedral, or tabular octahedral) known to be useful in photography.

Conventional vehicles can be used such as those described in paragraph IX of the above mentioned publication. Silver halides can be spectrally sensitized as described in paragraph IV.

The photographic elements according to the invention can contain optical brightening agents, antifoggants; stabilizing agents, absorbing or diffusing agents, coating aids, plasticizers, lubricants and matting agents as described in paragraphs V, VI, VIII, XI, XII, and XVI. Methods for adding components, coating and drying methods can be used, such as those described in paragraphs XIV and XV. Usual photographic supports can be used, such as those described in paragraph XVII. The resulting photographic elements can be for black and white photography, or preferably for color photography that form silver images and/or dye images by selective dye destruction, formation or physical elimination, as described in paragraph VII. Preferred color photographic elements are those that form dye images by means of color developers or dye forming couplers. To use these elements, they can be exposed in a usual manner, as described in paragraph XVIII, then they can be processed as described in paragraph XIX.

Still in another embodiment, the cyclic thioether as described is present in the processing composition in an amount of from $5.10^{-5}$ to $1.10^{-3}$ mole/liter. Here also the thioether acts as a development accelerator.

The following examples illustrate the invention.

EXAMPLE 1

A silver bromoiodide emulsion (1.8 % iodide) having three-dimensional grains of average diameter = 0.75 µm was prepared and chemically sensitized with:

| | | |
|---|---|---|
| $Na_2S_2O_3$ | = | 2.9 mg/mole Ag |
| $KAuCl_4$ | = | 0.6 mg/mole Ag |
| NaSCN | = | 43.8 mg/mole Ag |

The thioethers were introduced in a water-methanol solution after redispersion of the coagulum in gelatin at a concentration of $1.7.10^{-4}$ mole/mole Ag. The emulsion was heated to 60°C, then cooled to 40°C, at which temperature the components for the S + Au sensitization were added. The emulsion was then heated to 65°C. Samples were taken every five minutes then coated in the presence of glycerol at a coverage of 50 mg silver/dm².

The films were exposed for 1/50th s in a sensitometer X6 (2850°K, Wratten 39 filter) and developed, either 6 mn in the Kodak Developer D19b, or 8 mn in an Elon-ascorbic acid (EAA) surface developer.

The control used was: 1,10-dithia-4,7,13,16-tetraoxa-cyclooctadecane

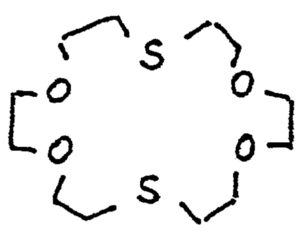

The sensitometric results are gathered in table I.

Table I

| Compounds | Concentration $10^{-4}$ mole/mole Ag | Heat spike duration at 65°C (mn) | Sensitometric characteristics Developer D 19b (6 mn at 20°C) | | | | | Developer EAA (8 mn at 20°C) | |
|---|---|---|---|---|---|---|---|---|---|
| | | | Gross fog | Log relative sensitivity | ΔLog E | Contrast | Reciprocity law failure (10"–1/10") | Gross fog | Log relative sensitivity |
| Control | 1.7 | 45 | 0.20 | 440 | 0 | 1.90 | −0.05 | 0.19 | 436 |
| 1 | 1.7 | 15 | 0.25 | 460 | +0.2 | 1.80 | +0.01 | 0.25 | 487 |
| 2 | 1.7 | 27 | 0.25 | 435 | −0.05 | 1.80 | +0.01 | 0.30 | 505 |
| 3 | 1.7 | 7 | 0.25 | 455 | +0.15 | 2.15 | −0.04 | 0.25 | 520 |
| 4 | 1.7 | 12 | 0.25 | 460 | +0.20 | 2.30 | −0.05 | 0.18 | 510 |
| 5 | 1.7 | 10 | 0.24 | 450 | +0.10 | 2.20 | +0.02 | 0.30 | 534 |
| 6 | 1.7 | 15 | 0.25 | 455 | +0.15 | 1.90 | 0 | 0.23 | 514 |

These results show that the compounds according to the invention give an improved sensitivity in comparison to the control with Kodak Developer D19b, with which only the sensitization effect appears.

With the surface developer EAA, the increase in sensitivity is still more observable, since in this developer the development accelerator effect appears in addition.

EXAMPLE 2

In a conventional layer Kodachrome film, the following amounts of 1,4,7-trioxa-10,13,16-trithiacyclooctadecane were introduced in the interlayers:

| | |
|---|---|
| UV filter layer | $8.70^{-7}$ mg/dm$^2$ |
| Magenta interlayer | $7.10^{-7}$ mg/dm$^2$ |
| Cyan interlayer | $1.3.10^{-6}$ mg/dm$^2$ |

The film was then exposed and developed in the usual manner. A control film without thioether was exposed and processed in the same manner.

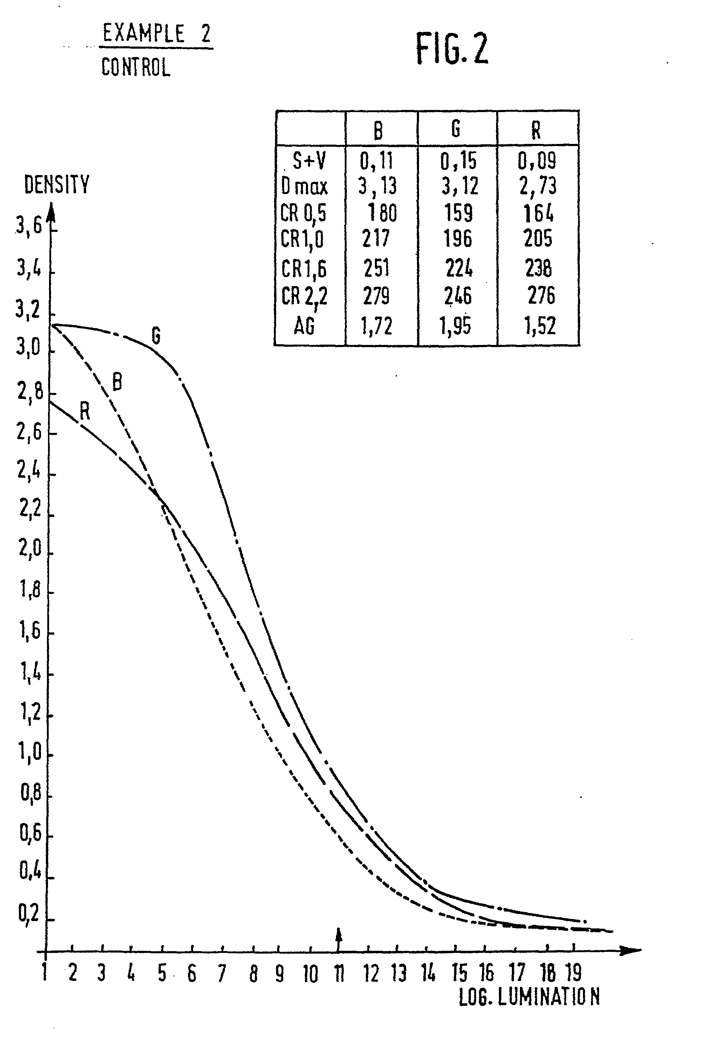

Figures 1 and 2 represent blue (B), green (G) and red (R) densities plotted as functions of log exposure. In the tables, S + V is the gross fog, i.e. the densitz of the support plus the fog in the unexposed areas; Dmax is the maximum density; the CR values are measurements in log of relative sensitivity; the values of the test sample of the fig. 1 have to be compared with the control sample of the fig. 2. AG is the silver laydown in mg/dm$^2$.

The curves and tables of Figs 1 and 2 show that the sensitivity of the thioether-containing film is markedly improved. The thioether here performs the function of a development accelerator.

1,10-dithia-4,7,13,16-tetraoxacyclooctadecane used in the same conditions does not give any improvement of the sensitivity in comparison to the control without thioether.

**Claims**

1. Photographic element comprising a support and at least one silver halide emulsion layer characterized in that it contains a cyclic thioether compound which comprises at least three sulfur atoms and at least one oxygen atom, each sulfur or oxygen atom being separated from another sulfur or oxygen atom by a polymethylene group.

2. Photographic element according to claim 1, characterized in that each sulfur or oxygen atom of the cyclic thioether compound is separated from any sulfur or oxygen atom by a group $-(CH_2)_n-$, wherein n is 2 or 3.

3. Photographic element according to any of claims 1 or 2, characterized in that said compound is 1,4-dioxa-7,10,13-trithiacyclopentadecane.

4. Photographic element according to any of claims 1 or 2, characterized in that said compound is 1-oxa-6,9,12-trithiacyclohexadecane.

5. Photographic element according to any of claims 1 or 2, characterized in that said compound is 1,7,13-trioxa-4,10,16-trithiacyclooctadecane.

6. Photographic element according to any of claims 1 or 2, characterized in that said compound is 1,4,7-trioxa-10,13,16-trithiacyclooctadecane.

7. Photographic element according to any of claims 1 or 2, characterized in that said compound is 1,4,7,10-tetraoxa-13,16,19-trithiacycloheneicosane.

8. Photographic element according to any of claims 1 or 2, characterized in that said compound is 1,10-dioxa-4,7,13,16-tetrathiocyclooctadecane.

9. Photographic element according to any of claims 1 to 8, characterized in that said compound is present in at least one silver halide emulsion layer.

10. Photographic element according to claim 9, characterized in that said compound is present in the emulsion layer in an amount of from $1.10^{-4}$ to $5.10^{-4}$ mole/silver mole.

11. Photographic element according to any of claims 1 to 8, characterized in that said compound is present in another layer than an emulsion layer.

12. Photographic element according to claim 11, characterized in that said compound is present in said layer at a coverage of from $1.10^{-7}$ to $1.10^{-6}$ mg/dm$^2$.

13. Composition for developing silver halides, characterized in that said composition contains a cyclic thioether compound which comprises at least three sulfur atoms and at least one oxygen atom, each sulfur or oxygen atom being separated from another sulfur or oxygen atom by a polymethylene group.

14. Developing composition according to claim 13, characterized in that the concentration of said compound in the composition is in the range from $5.10^{-5}$ to $1.10^{-3}$ mole/liter.

**Revendications**

1. Produit photographique comprenant un support et au moins une couche d'émulsion aux halogénures d'argent, caractérisé en ce qu'il contient un composé thioéther cyclique, qui comprend au moins trois atomes de soufre et au moins un atome d'oxygène, chaque atome de soufre ou d'oxygène étant séparé d'un autre atome de soufre ou d'oxygène par un groupe polyméthylène.

2. Produit photographique selon la revendication 1, caractérisé en ce que chaque atome de soufre ou d'oxygène du composé thioéther cyclique est séparé d'un quelconque atome de soufre ou d'oxygène par un groupe $-(CH_2)-_n$, où n est égal à 2 ou 3.

3. Produit photographique selon l'une quelconque des revendications 1 ou 2, caractérisé en ce que ledit composé est le 1,4-dioxa-7,10,13-trithiacyclopentadécane.

4. Produit photographique selon l'une quelconque des revendications 1 ou 2, caractérisé en ce que ledit composé est le 1-oxa-6,9,12-trithiacyclohexadécane.

5. Produit photographique selon l'une quelconque des revendications 1 ou 2, caractérisé en ce que ledit composé est le 1,7,13-trioxa-4,10,16-trithiacyclooctadécane.

6. Produit photographique selon l'une quelconque des revendications 1 ou 2, caractérisé en ce que ledit composé est le 1,4,7-trioxa-10,13,16-trithiacyclooctadécane.

7. Produit photographique selon l'une quelconque des revendications 1 ou 2, caractérisé en ce que ledit composé est le 1,4,7,10-tétraoxa-13,16,19-trithiacycloheneicosane.

8. Produit photographique selon l'une quelconque des revendications 1 ou 2, caractérisé en ce que ledit composé est le 1,10-dioxa-4,7,13,16-tétrathiocyclooctadécane.

9. Produit photographique conforme à l'une quelconque des revendications 1 à 8, caractérisé en ce que ledit composé est présent dans au moins une couche d'émulsion aux halogénures d'argent.

10. Produit photographique conforme à la revendication 9, caractérisé en ce que ledit composé est présent dans la couche d'émulsion à raison de $1.10^{-4}$ à $5.10^{-4}$ mole/mole d'argent.

11. Produit photographique conforme à l'une quelconque des revendications 1 à 8, caractérisé en ce que ledit composé est présent dans une autre couche qu'une couche d'émulsion.

12. Produit photographique conforme à la revendication 11, caractérisé en ce que ledit composé est présent dans ladite couche à raison de $1.10^{-7}$ à $1.10^{-6}$ mg/dm$^2$.

13. Composition de développement des halogénures d'argent caractérisée en ce qu'elle contient un composé thioéther cyclique qui comprend au moins trois atomes de soufre et au moins un atome d'oxygène, chaque atome de soufre ou d'oxygène étant séparé d'un autre atome de soufre ou d'oxygène par un groupe polyméthylène.

14. Composition de développement conforme à la revendication 13, caractérisée en ce que la concentration dudit composé dans la composition est comprise entre $5.10^{-5}$ et $1.10^{-3}$ mole/litre.

**Patentansprüche**

1. Photographisches Element mit einem Träger und mindestens einer Silberhalogenidemulsionsschicht, dadurch gekennzeichnet, daß es eine cyclische Thioetherverbindung enthält, die mindestens drei Schwefelatome und mindestens ein Sauerstoffatom aufweist, wobei jedes Schwefel- oder Sauerstoffatom von einem anderen Schwefel- oder Sauerstoffatom durch eine Polymethylengruppe getrennt ist.

2. Photographisches Element nach Anspruch 1, dadurch gekennzeichnet, daß jedes Schwefel- oder Sauerstoffatom der cyclischen Thioetherverbindung von einem anderen Schwefel- oder Sauerstoffatom durch eine Gruppe $-(CH)_2)_n-$, in der n für 2 oder 3 steht, getrennt ist.

3. Photographisches Element nach einem der Ansprüche 1 oder 2, dadurch gekennzeichnet, daß die Verbindung aus 1,4-Dioxa-7,10,13-trithiacyclopentadecan besteht.

4. Photographisches Element nach einem der Ansprüche 1 oder 2, dadurch gekennzeichnet, daß die Verbindung aus 1-Oxa-6,9,12-trithiacyclohexadecan besteht.

5. Photographisches Element nach einem der Ansprüche 1 oder 2, dadurch gekennzeichnet, daß die Verbindung aus 1,7,13,Trioxa-4,10,16-trithiacyclooctadecan besteht.

6. Photographisches Element nach einem der Ansprüche 1 oder 2, dadurch gekennzeichnet, daß die Verbindung aus 1,4,7-Trioxa-10,13,16-trithiacyclooctadecan besteht.

7. Photographisches Element nach einem der Ansprüche 1 oder 2, dadurch gekennzeichnet, daß die Verbindung aus 1,4,7,10-Tetraoxa-13,16,19-trithiacycloheneicosan besteht.

8. Photographisches Element nach einem der Ansprüche 1 oder 2, dadurch gekennzeichnet, daß die Verbindung aus 1,10-Dioxa-4,7,13,16-tetrathiocyclooctadecan besteht.

9. Photographisches Element nach einem der Ansprüche 1 bis 8, dadurch gekennzeichnet, daß die Verbindung in mindestens einer Silberhalogenidemulsionsschicht vorliegt.

10. Photographisches Element nach Anspruch 9, dadurch gekennzeichnet, daß die Verbindung in der Emulsionsschicht in einer Menge von $1.10^{-4}$ bis $5.10^{-4}$ Mol/Mol Silber vorliegt.

11. Photographisches Element nach einem der Ansprüche 1 bis 8, dadurch gekennzeichnet, daß die Verbindung in einer anderen Schicht als einer Emulsionsschicht vorliegt.

12. Photographisches Element nach Anspruch 11, dadurch gekennzeichnet, daß die Verbindung in dieser Schicht in einer Beschichtungsstärke von $1.10^{-7}$ bis $1.10^{-6}$ $mg/dm^2$ vorliegt.

13. Zusammensetzung für die Entwicklung von Silberhalogeniden, dadurch gekennzeichnet, daß die Zusammensetzung eine cyclische Thioetherverbindung enthält, die mindestens drei Schwefelatome und mindestens ein Sauerstoffatom aufweist, wobei jedes Schwefel- oder Sauerstoffatom von einem anderen Schwefel- oder Sauerstoffatom durch eine Polymethylengruppe getrennt ist.

14. Entwicklungszusammensetzung nach Anspruch 13, dadurch gekennzeichnet, daß die Konzentration dieser Verbindung in der Zusammensetzung im Bereich von $5.10^{-5}$ bis $1.10^{-3}$ Mol/Liter liegt.

# FIG.1

EXAMPLE 2

FILM CONTAINING
1,4,7-trioxa-10,13,16-trithiacyclooctadecane.

| | B | G | R |
|---|---|---|---|
| S+V | 0,09 | 0,13 | 0,07 |
| D max | 2,85 | 2,98 | 2,57 |
| CR0,5 | 207 | 189 | 204 |
| CR 1,0 | 241 | 217 | 231 |
| CR1,6 | 270 | 239 | 258 |
| CR 2,2 | 298 | 257 | 292 |
| AG | 1,87 | 2,52 | 1,93 |

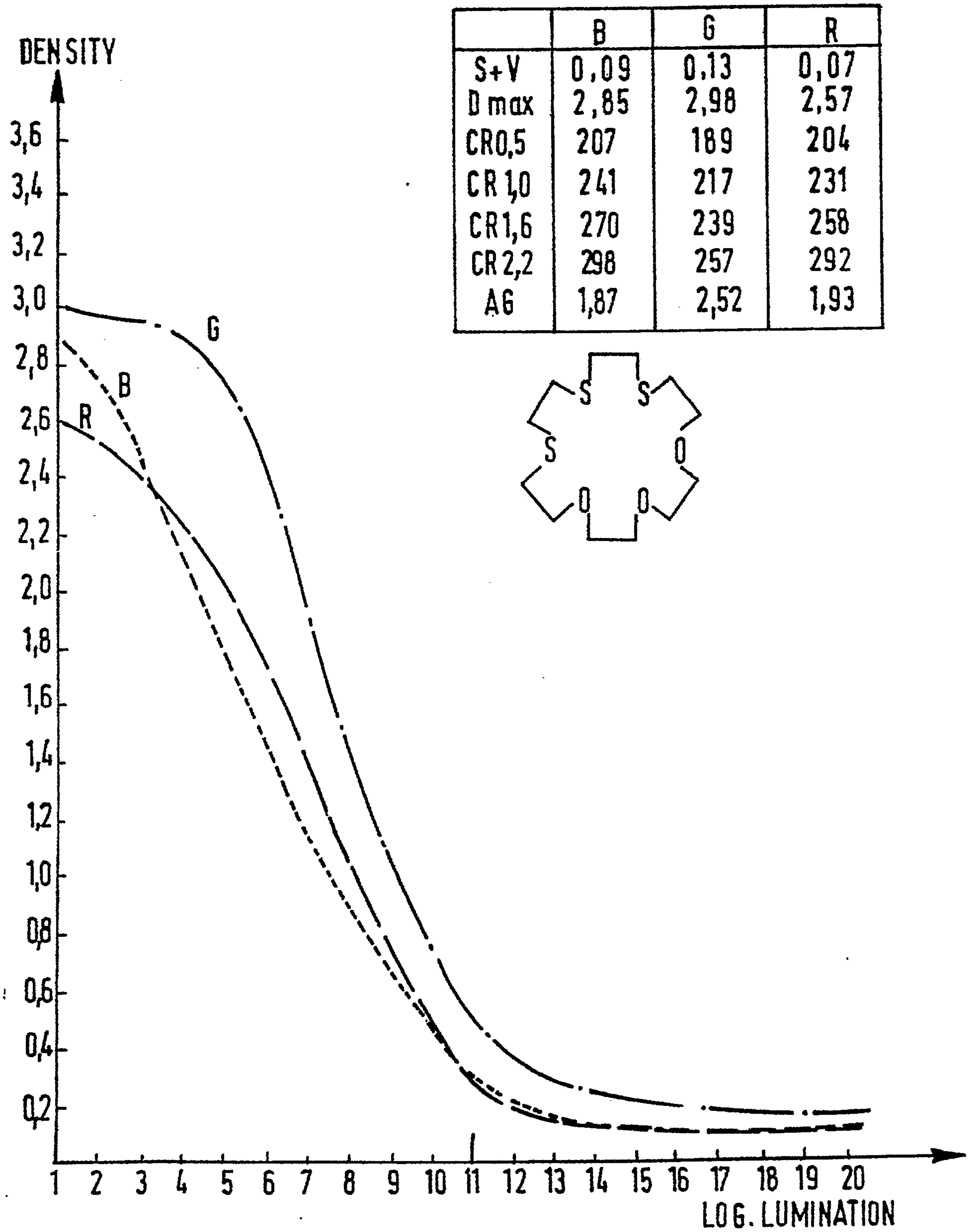

EXAMPLE 2

CONTROL

FIG. 2

| | B | G | R |
|---|---|---|---|
| S+V | 0,11 | 0,15 | 0,09 |
| D max | 3,13 | 3,12 | 2,73 |
| CR 0,5 | 180 | 159 | 164 |
| CR1,0 | 217 | 196 | 205 |
| CR1,6 | 251 | 224 | 238 |
| CR 2,2 | 279 | 246 | 276 |
| AG | 1,72 | 1,95 | 1,52 |